(19) Europäisches Patentamt
European Patent Office
Office européen des brevets

(11) Veröffentlichungsnummer: **0 629 402 A1**

# (12) EUROPÄISCHE PATENTANMELDUNG

(21) Anmeldenummer: **94108571.4**

(22) Anmeldetag: **03.06.94**

(51) Int. Cl.[5]: **A61K 31/505**, A61K 9/20, A61K 9/28, A61K 9/50

(30) Priorität: **15.06.93 DE 4319760**

(43) Veröffentlichungstag der Anmeldung: **21.12.94 Patentblatt 94/51**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Serno, Peter, Dr.**
**Offenbachstrasse 12**
**D-51467 Bergisch Gladbach (DE)**
Erfinder: **Fischer, Wolfgang, Dr.**
**Gottfried-Kinkel-Strasse 51**
**D-53721 Siegburg (DE)**
Erfinder: **Rupp, Roland, Dr.**
**Haferbusch 21**
**D-51467 Bergisch Gladbach (DE)**
Erfinder: **Versavel, Mark, Dr.**
**Karlrobert-Kreiten-Strasse 4**
**D-40724 Hilden (DE)**
Erfinder: **Tenter, Ulrich, Dr.**
**Hossenhauser Strasse 123**
**D-42655 Solingen (DE)**
Erfinder: **Schmidt, Gerhard, Dr.**
**Holunderweg 32**
**D-51107 Köln (DE)**
Erfinder: **Schöllnhammer, Günter, Dr.**
**Hackberg 21**
**D-51429 Bergisch Gladbach (DE)**
Erfinder: **Ammen, Maria**
**Hoffnungsthaler Strasse 18**
**D-51503 Rösrath (DE)**
Erfinder: **Maegata, Shinji**
**833-147, Ohara-naka,**
**Koka-cho**
**Koka-gun,**
**Shiga (JP)**

## (54) Ipsapiron Arzneimittelzubereitung.

(57) Die Erfindung betrifft Ipsapiron-Arzneimittelzubereitungen mit retardierter Wirkung, hoher Bioverfügbarkeit und guter Lagerstabilität bestehend aus diffusionskontrollierten Pellets oder Erosionstabletten mit definierter Wirkstofffreisetzung.

EP 0 629 402 A1

Die Erfindung betritt eine Ipsapiron Arzneimittelzubereitung mit retardierter Wirkung und hoher Lagerstabilität, Verfahren zur Herstellung und ihre Verwendung.

Ipsapiron ist eine neue Wirksubstanz, die therapeutisch gegen Angstzustände und Depressionen eingesetzt wird.

Als Ergebnis pharmakokinetischer Untersuchungen wurde festgestellt, daß Ipsapiron nach oraler Gabe eine Bioverfügbarkeit von nur 40 % aufweist. Ein erheblicher Wirkstoffanteil wird bereits präsystemisch metabolisiert und kann nicht bioverfügbar werden. Dies ist nachteilig, weil dieser Substanzverlust durch eine erhöhte Wirkstoffdosis ausgeglichen werden muß. Es resultieren große, für den Patienten schlecht schluckbare Arzneiformen und eine erhebliche Belastung des Patienten mit pharmakologisch inaktiven Ipsapiron-Metaboliten. Ferner nehmen mit sinkender Bioverfugbarkeit die interindividuellen Schwankungen im Plasmakonzentrationsverlauf zu und die Therapie wird unkalkulierbar.

Ein weiterer pharmakokinetischer Nachteil von Ipsapiron liegt in der kurzen Eliminationshalbwertszeit. Als Folge müssen Ipsapiron-Tabletten mindestens dreimal täglich eingenommen werden. Dies hat insbesondere für berufstätige Patienten eine Reihe von Unannehmlichkeiten zur Folge, es besteht ein hohes Risiko des Vergessens einer Tabletteneinnahme, und die Bereitschaft und Fähigkeit von Patienten eine solche Therapie über lange Zeit korrekt einzuhalten sind gering.

Es besteht somit ein erheblicher Bedarf an einer Arzneiform für Ipsapiron, die die ungünstigen Substanzeigenschaften kompensiert, also die Bioverfügbarkeit des Wirkstoffes steigert <u>und</u> gleichzeitig eine Verringerung der Einnahmefrequenz erlaubt.

Wirksame und therapeutisch vertretbare Methoden zur Verminderung der präsystemischen Metabolisierung von Ipsapiron und somit Erhöhung der Bioverfügbarkeit sind bislang nicht bekannt. Absorptionsförderer wie sie beispielsweise in EP 0 206 947 für andere Wirkstoffe beschrieben werden, vermögen zwar eine unzureichende Absorption zu erhöhen, können aber eine extensive präsystemische Metabolisierung nicht vermindern. Die in WO 92/06680 beschriebene Formulierung in C12-C24-Fettsäuren zur Verminderung der first-pass-Metabolisierung erfordert eine ausreichende Löslichkeit des Wirkstoffes in Fettsäuren, die für Ipsapiron nicht gegeben ist. Ferner wird der Patient einer erheblichen Belastung mit Fettsäuren ausgesetzt.

Methoden zur Retardierung der Freisetzung pharmakologisch aktiver Wirkstoffe und somit Reduzierung der Einnahmefrequenz sind dem Fachmann hinreichend bekannt. Allerdings tritt bei allen üblichen Methoden der Retardierung ein mehr oder weniger starker Verlust an Bioverfügbarkeit ein, die im Fall von Ipsapiron ohnehin bereits kritisch ist. Retardierung <u>und</u> Erhöhung der Bioverfügbarkeit sind nach dem gegenwärtigen Stand der Technik zwei gegenläufige Anforderungen, zu deren gleichzeitigen Erreichen keine Methoden bekannt sind.

Es wurde nun gefunden, daß die Bioverfügbarkeit von Ipsapiron bei gleichzeitiger Retardierung dadurch erhöht werden kann, daß der Wirkstoff in eine Arzneiform überführt wird, welche den Wirkstoff mit einer mittleren Freisetzungsrate von 80 % der Dosis/5 Stunden bis 80 % der Dosis/13 Stunden abgibt <u>und</u> deren initiale Freisetzungsrate in den ersten beiden Stunden der Freisetzung weniger als 35 % beträgt. Überraschenderweise zeigen die erfindungsgemäßen Ipsapiron Arzneimittelformen eine Erhöhung der Bioverfügbarkeit um beispielsweise 24 %, wobei gleichzeitig der Plasmakonzentrationsverlauf so verzögert wird, daß die Einnahmefrequenz auf einmal pro Tag abgesenkt werden kann.

Dies ist umso überraschender, als nach bisherigen Kenntnissen davon auszugehen ist, daß die Bioverfügbarkeit von Wirkstoffen durch eine kontrollierte, langsame Freisetzung eher abnimmt. So faßt SKELLY (Skelly, J., Regulatory Recommendations in USA on Investigation and Evaluation of Oral Controlled Release Products. in: Gundert-Remy, Möller, Oral Controlled Release Products, Wiss. Verlagsgesellschaft Stuttgart 1990, Seite 180) zusammen: "many controlled release products are somewhat less bioavailable than their immediate release counterparts." Speziell für den Fall von Wirkstoffen mit hoher präsystemischer Metabolisierung beschreibt WAGNER (Wagner, J.G., Oral Sustained Release and Prolonged-Action Medication. in: Fundamentals of Clinical Pharmacokinetics. Hamilton Drug Intelligence Publ. 1975, Seite 149) den Stand der Technik wie folgt: "if a drug is metabolized in the gastrointestinal barrier during absorption then it is feasible that a greater percentage of the dose in a slow release form will be metabolized than when the same dose is administered in a fast release form." Ferner überwinden die erfindungsgemäßen Zubereitungen das bisherige Vorurteil, "daß nach etwa 7 Stunden der Abschluß der Freisetzung des Arzneistoffes erreicht sein muß. Andernfalls ist mit Beeinträchtigung des Bioverfügbarkeitsausmaßes zu rechnen" (Lippold, B.C., Biopharmazie. Wiss. Verlagsgesellschaft Stuttgart 1984, Seite 69).

Zur Herstellung der erfindungsgemäßen Ipsapiron-Arzneimittelzubereitung sind unterschiedliche Verfahren der Wirkstoffretardierung einsetzbar, doch muß eine Freisetzungsgeschwindigkeit des erfindungsgemäßen Bereiches erzielt werden.

Zur Ermittlung der erfindungsgemäß geforderten initialen und mittleren Freisetzungsrate werden die Arzneiformen in der "Apparatur 2" der USP XXII geprüft (The United States Pharmacopeia USP XXII 1990,

Seite 1578). Als Testmedium wird 900 ml 0.1 molare Salzsäure verwendet. Die Umdrehungsgeschwindigkeit des Rührers beträgt 75 Umdrehungen pro Minute. Proben werden durch einen 8 µm Filter gezogen und deren Wirkstoffgehalt per HPLC bestimmt. Die auf diese Weise als aufgelöst bestimmte Wirkstoffmenge wird in Prozent der eingesetzten Wirkstoffmenge umgerechnet.

Bevorzugte erfindungsgemäße Arzneimittelformulierungen sind diffusionskontrollierte Pellets, Erosionstabletten oder osmotische Abgabesysteme von Ipsapiron wie Tabletten oder Pellets mit Membranen kontrollierter Porosität oder Dreischichterosionstabletten, in denen lediglich die mittlere Schicht Wirkstoff enthält.

Die erfindungsgemäßen diffusionskontrollierten Pellets bestehen im allgemeinen aus Neutralpellets auf die eine Mischung des Wirkstoffs mit üblichen Binde- und Verdickungsmitteln, gegebenenfalls gemeinsam mit Zusatzstoffen, aufgetragen wird und die anschließend mit einem üblichen Diffusionslack, enthaltend übliche Weichmacher, überzogen werden.

Als Binde- und Verdickungsmittel, wird bevorzugt Hydroxypropylmethylcellulose verwendet. Ebenso können auch andere natürliche, synthetische oder halbsynthetische Polymere wie beispielsweise Methylcellulose, Hydroxypropylcellulose, Natriumcarboxymethylcellulose, Polyacrylsäuren oder Gelatine eingesetzt werden.

Als Diffusionslack eignet sich bevorzugt Aquacoat®, aber auch andere Materialien wie Acrylate, Celluloseacetat, Celluloseacetatbutyrat, Surelease® oder Ethylcellulose in Form weiterer wässriger Dispersionen oder Lösungen.

Als Weichmacher eignet sich bevorzugt Triacetin, es können jedoch auch Dibutylsebacat, mittelkettige Triglyceride, acetylierte Monoglyceride, Triethylcitrat, Acetyltributylcitrat, Diethylphtalat oder Dibutylphtalat als Weichmacher eingesetzt werden. Da Art und Menge des Weichmachers die Freisetzungsrate beeinflussen, muß die Beschichtungsmenge an Diffusionslack gegebenenfalls so verändert werden, daß Pellets der erfindungsgemäßen Freisetzungsrate resultieren.

Bei den erfindungsgemäßen Pellets ist es insbesondere wichtig, ein bestimmtes Verhältnis von wirkstoffüberzogenen Pellets zur Diffusionsmembran sowie ein bestimmtes Verhältnis von Lacktrockensubstanz zu Weichmachermenge zu verwenden.

Bevorzugt beträgt bei Verwendung von Neutralpellets einer Größe von 0,85 - 1 mm, einem Verhältnis von Neutralpellets zu Wirkstoff (berechnet als wasserfreies Ipsapironhydrochlorid) von 40 : 10, einem Verhältnis von Bindemittel, insbesondere HPMC, zu Wirkstoff berechnet als wasserfreies Ipsapironhydrochlorid von 5 : 10 und einem Verhältnis von physiologisch verträglicher Säure, insbesondere Kaliumhydrogentartrat, zu Wirkstoff (berechnet als wasserfreies Ipsapironhydrochlorid) von 10 : 10

das Verhältnis der Masse wirkstoffüberzogener Pellets zur Masse an Diffusionslack (ohne Weichmacher) 100 : 6 bis 100 : 21

und das Verhältnis der Masse an Diffusionslack zur eingesetzten Masse an Weichmacher 100 : 5 bis 100 : 60, bevorzugt 100 : 20 bis 100 : 45.

Es ist zu berücksichtigen, daß Teile des eingesetzten Weichmachers während des Lackierens und Nachtemperns abdunsten können. So werden nach Auftrag von 8,5 % Aquacoat®-Trockensubstanz beispielsweise nur noch etwa 86 % der theoretischen Triacetin-Menge gefunden, nach einstündigem Nachtempern bei 70 °C in der Wirbelschicht nur noch etwa 15 % der theoretischen Triacetin-Menge.

Bei Änderung der genannten Randbedingungen ist eine Veränderung der erfindungsgemäßen Beschichtungsmenge an Diffusionslack erforderlich. So ist beispielsweise eine höhere Beschichtungsmenge erforderlich, wenn die relative Neutralpelletmenge erhöht wird, die Menge Hydroxypropylmethylcellulose erhöht wird oder der Triacetin-Anteil verringert wird. Eine niedere Beschichtungsmenge ist erforderlich, wenn die relative Neutralpelletmenge erniedrigt wird, die Menge Hydroxypropylmethylcellulose erniedrigt wird oder der Triacetinanteil erhöht wird.

Die erfindungsgemäßen Diffusionspellets können beispielsweise hergestellt werden, indem man mikrofeines Ipsapironhydrochioridhydrat und gegebenenfalls eine physiologisch verträgliche Säure in Wasser suspendiert bzw. löst und mit einer konzentrierten Methylhydroxypropylcellulose-Lösung verdickt. Die so erhaltene Suspension wird in einer Wirbelschichtanlage in einem Sprühprozeß auf Neutralpellets aufgezogen. Es folgt die Beschichtung der Pellets mit einer Diffusionsmembran durch Aufsprühen beispielsweise einer wäßrigen Ethylcellulosedispersion, die einen geeigneten, physiologisch verträglichen Weichmacher enthält. Die Pellets werden anschließend bei Temperaturen von 50-125 °C, vorzugsweise 60-110 °C getempert. Dabei führen höhere Temperaturen der Temperung dazu, daß zur Erzielung der erfindungsgemäßen Freisetzungsrate eher niedere Lackauftragsmengen ausreichen und die entstehenden Pellets bei Lagerung physikalisch stabiler sind. Die Dicke der Diffusionsmembran, Weichmachertyp, Weichmachermenge und Pelletgröße werden so gewählt, daß eine Freisetzungsgeschwindigkeit von 80 % des Ipsapiron in 5-13 Stunden resultiert und in den ersten beiden Stunden weniger als 35 % der Dosis abgegeben wird. Die einer

Tagesdosis von beispielsweise 10 mg, 20 mg, 30 mg oder 40 mg Ipsapiron entsprechende Menge Pellets wird in eine Hartgelatinekapsel gefüllt.

Neben der beschriebenen Beschichtung von Neutralpellets sind auch andere Methoden der Pelletherstellung gangbar wie das Extrusions-/Spheronizer-Verfahren, die Rotorgranulation oder die Tablettierung.

Außerdem bevorzugt sind erfindungsgemäße Arzneizubereitungen in Form von Erosionstabletten. Diese Tabletten sind dadurch gekennzeichnet, daß sie neben üblichen Hilfs- und Trägerstoffen sowie Tablettierhilfsstoffen, eine bestimmte Menge an wasserlöslichen, hydrogelbildenden Polymeren enthalten, wobei diese Polymere eine Viskosität von mindestens 150 mPa • s (gemessen als 2 %ige wäßrige Lösung bei 20 °C) haben müssen.

Übliche Hilfs- und Trägerstoffe sind beispielsweise Laktose, mikrokristalline Cellulose, Mannit oder Calciumphosphate.

Übliche Tablettierhilfsmittel sind beispielsweise Magnesiumstearat, Talkum oder hochdisperses Siliciumdioxid (Aerosil®).

Als wasserlösliche, hydrogelbildende Polymere werden bevorzugt Hydroxypropylcellulose, aber auch Hydroxypropylmethylcellulosen, Methylcellulosen, Carboxymethylcellulose, Alginate, Galaktomannane, Polyacrylsäure, Polymethacrylsäuren oder Copolymerisate aus Methacrylsäure und Methylmethacrylat eingesetzt.

Besonders bevorzugt ist die Verwendung von Hydroxypropylcellulose.

Hierbei sollten die erfindungsgemäßen Erosionstabletten mindestens 50 mg eines Hydroxypropylcellulosetyps bezogen auf die Masse einer Tablette enthalten, dessen Viskosität (gemessen als 2 %ige wäßrige Lösung bei 20 °C) mindestens 150 mPa • s beträgt.

Diese Angaben gelten für Hydroxypropylcellulosen mit einem Hydroxypropoxylgruppen-Anteil von 53-78 % und einer Partikelgröße von 99 % < 150 µm, 20-35 % 150-63 µm, 50-60 % 63-38 µm und 15-25 % < 38 µm. Bei Abweichung davon ist eine Anpassung der erfindungsgemäßen Menge erforderlich.

Die erfindungsgemäßen Tabletten können beispielsweise hergestellt werden, indem man Ipsapironhydrochloridhydrat in einer Hydroxypropylcelluloselösung suspendiert. Die Suspension wird verwendet, um eine Pulvermischung hydrophiler Gelbildner, beispielsweise Hydroxypropylcellulosen einer oder mehrerer Viskositätsstufen, im Wirbelschichtgranulator zu granulieren. Dabei wird die Menge und Viskositätsstufe der Hydroxypropylcellulose(n) so gewählt, daß Tabletten der erfindungsgemäßen mittleren Freisetzungsgeschwindigkeit und initialen Freisetzungsrate resultieren. Das trockene Granulat wird gesiebt, gegebenenfalls mit einer physiologisch verträglichen Säure und danach mit einem Schmiermittel wie Magnesiumstearat vermischt, tablettiert und gegebenenfalls noch lackiert.

Ein weiteres, erhebliches Problem bei der Formulierung von Ipsapiron stellt dessen Instabilität dar. Unter äußeren Einflüssen wie Temperatur und Feuchte reagiert der Wirkstoff wie folgt:

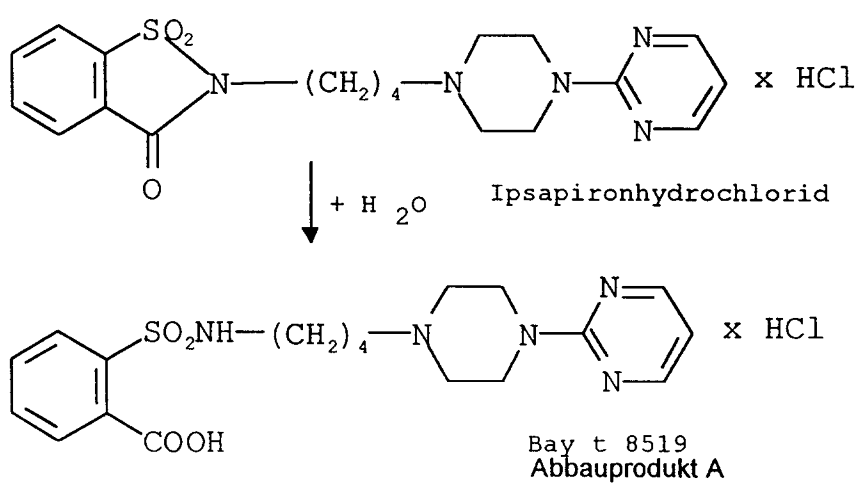


Ipsapironhydrochlorid

Bay t 8519
Abbauprodukt A

Die Reaktionsgeschwindigkeit ist dabei sehr erheblich. So wurde gefunden, daß eine Lösung von 6,7 mg Ipsapironhydrochlorid in 100 ml Phosphatpuffer pH 6.8 nach 24 Stunden bei 37 °C sich bereits zu 29 % zersetzt hat. Diese Reaktion kann auch in langsam freisetzenden Arzneiformen stattfinden, so daß während der Magen-Darm-Passage Anteile des Wirkstoffes abgebaut werden, bevor sie zur Resorption gelangen. Als Folge tritt eine erniedrigte Bioverfügbarkeit ein.

Dieselbe Abbaureaktion führt auch zu Instabilität bei Lagerung. So zeigt die Tablette des Vergleichsbeispiels 1 nach einer Lagerung über 6 Monate bei 40 °C zu 2,23 % (bez. auf Wirkstoffgehalt) das Abbauprodukt A.

Es wurde nun gefunden, daß diese Abbaureaktion ohne andersartige Nachteile vermieden werden kann, wenn der Ipsapiron-Arzneiform eine physiologisch verträgliche Säure oder sauer reagierende Salze wie Kaliumhydrogentartrat, Adipinsäure, Ascorbinsäure, Asparaginsäure, Benzoesäure, Citronensäure, Fumarsäure, Glutaminsäure, Maleinsäure, Natriumdihydrogencitrat, Natriumdihydrogenphosphat, Polyacrylsäure, Sorbinsäure, Stearinsäure, Bernsteinsäure, Weinsäure oder Kombinationen davon zugesetzt werden. Besonders bevorzugt sind dabei Säuren mit einer Löslichkeit in Wasser von weniger als 10 % (bei 20 °C) wie Adipinsäure, Asparaginsäure, Benzoesäure, Fumarsäure, Glutaminsäure, Kaliumhydrogentartrat, Bernsteinsäure und Stearinsäure.

Die Ergebnisse der Untersuchung der Bioverfügbarkeit (Tabelle 1) zeigen, daß dadurch erhebliche Einbrüche der Bioverfügbarkeit gegenüber der schnell freisetzenden Tablette (Beispiel Nr. 1) in allen Fällen (Beispiele Nr. 2-6) vermieden werden konnten. Ferner wurden Kapseln mit Pellets des Beispiels Nr. 6 über 6 Monate bei 40 °C eingelagert. Dabei wurde das Abbauprodukt A nur noch zu 0,22 % gebildet, was einer Reduktion auf ein Zehntel des Wertes der nicht stabilisierten Formulierung des Vergleichbeispiels 1 entspricht.

Zur Demonstration der Effektivität der erfindungsgemäßen Arzneiform wurden erfindungsgemäße und andere Retardarzneiformen von Ipsapiron jeweils 6-8 freiwilligen, gesunden Probanden verabreicht und Dauer des Plasmaspiegels sowie Bioverfügbarkeit im Vergleich zu einer schnell freisetzenden Ipsapiron-Tablette gemessen (Abbildung 1).

Die erfindungsgemäße Zubereitung des Beispiels Nr. 6 zeigt die überraschende Erhöhung der Bioverfügbarkeit von Ipsapiron um 24,4 % im Vergleich zu einer nicht retardierten Standardtablette. Wie Fig. 1 zeigt, wird beispielsweise eine Plasmakonzentration von 2 µg/l über 24 Stunden nach einer einzigen Kapsel-Einnahme aufrechterhalten.

Die Retardzubereitungen aus Beispiel 2 und 3 erfüllen die erfindungsgemäßen Kriterien nicht. Deren Bioverfügbarkeit liegt entsprechend dem bislang beschriebenen Stand der Technik mit Werten von 78 und 85 % unter der Bioverfügbarkeit der schnell freisetzenden Tablette.

Die Zubereitungen aus Beispiel 4 und 5 liegen in ihren Freisetzungseigenschaften im Grenzbereich der erfindungsgemäßen Kriterien. Dementsprechend ist deren Bioverfügbarkeit zwar noch erhöht, aber in geringerem Umfang.

Vergleicht man die erfindungsgemäße Retardzubereitung aus Beispiel 6 mit einer üblichen Retardzubereitung entsprechend dem bislang bekannten Stand der Technik (Beispiel 2), so gelang eine für die Therapie außerordentlich bedeutende Erhöhung der Bioverfügbarkeit um fast 60 %.

**Beispiele:**

**Beispiel 1**

**Schnell freisetzende Tablette / Stand der Technik**

Zusammensetzung pro Tablette:

| GRANULAT: | |
|---|---|
| Ipsapiron hydrochlorid | 10,0 mg |
| Lactose | 107,6 mg |
| Avicel | 48,0 mg |
| Maisstärke | 11,0 mg |
| Hydroxypropylmethylcellulose (HPMC) | 2,8 mg |

| NACHMISCHUNG: | |
|---|---|
| Magnesiumstearat | 0,6 mg |

| LACKHÜLLE: | |
|---|---|
| HPMC | 3,3 mg |
| Polyethylenglykol (PEG) | 1,1 mg |
| Titandioxid | 1,1 mg |

Ipsapironhydrochlorid, Lactose, Avicel und Maisstärke werden pulverförmig gemischt und mit wäßriger HPMC-Lösung granuliert. Das Granulat wird getrocknet, gesiebt und mit Magnesiumstearat gemischt. Es folgt die Verpressung zu Tabletten und Lackierung mit einer wäßrigen Dispersion von Titandioxid, PEG und HPMC.

**Beispiel 2**

**Retardtablette / Kriterien der Erfindung nicht erfüllend**

Zusammensetzung pro Tablette:

| GRANULAT: | |
|---|---|
| Ipsapironhydrochloridhydrat | 10,62 mg |
| HPC 8 mPa • s | 109,7 mg |
| HPC 300 mPa • s | 45,0 mg |
| Bernsteinsäure | 5,0 mg |

| NACHMISCHUNG: | |
|---|---|
| Magnesiumstearat | 0,3 mg |

| LACKHÜLLE: | |
|---|---|
| HPMC | 3,3 mg |
| PEG 4000 | 1,1 mg |
| Titandioxid | 1,1 mg |

Ipsapironhydrochloridhydrat wird in einer wäßrigen Lösung eines HPC 300 mPa • s-Anteils suspendiert. Das restliche HPC 300 mPa • s und HPC 8 mPa • s werden im Wirbelschichtgranulator mit dieser Suspension granuliert. Das Granulat wird getrocknet, gesiebt, mit Magnesiumstearat gemischt, zu Tabletten von 8 mm Durchmesser verpreßt und lackiert.

Die initiale Freisetzungsrate der Arzneiform beträgt 40 % in 2 Stunden, die mittlere Freisetzungsrate 80 % in 6 Stunden.

**Beispiel 3**

**Retardpellet / Kriterien der Erfindung nicht erfüllend**

Zusammensetzung pro Kapsel:

PELLETKERN:

| | |
|---|---|
| Neutralpellets 0,85 - 1,00 mm | 40,0 mg |
| Ipsapironhydrochloridhydrat | 10,62 mg |
| Kaliumhydrogentartrat | 10,0 mg |
| HPMC | 5,0 mg |

DIFFUSIONSMEMBRAN:

| | |
|---|---|
| AQUACOAT® ECD30-Trockensubstanz | 15,09 mg |
| Triacetin, eingesetzte Menge | 5,03 mg |

| | |
|---|---|
| Hartgelatinekapsel | |
| Kapsel Gr. 4 | 39,0 mg |

Ipsapironhydrochloridhydrat und Kaliumhydrogentartrat werden in wäßriger HPMC-Lösung suspendiert und in der Wirbelschicht auf Neutralpellets aufgesprüht. Eine wäßrige Aquacoat®-Triacetin-Dispersion wird aufgesprüht. Die Masse der aufgesprühten Diffusionsmembran beträgt (berechnet als Aquacoat®-Trockensubstanz) 23 % der Masse der nicht umhüllten Pellets. Der Aufsprühprozeß wird dreimal unterbrochen und die Pellets jeweils 1 Stunde bei 60 °C getrocknet. Die überzogenen Pellets werden über 1 Stunde bei 60 °C nachgetempert und in Hartgelatinekapseln abgefüllt.

Die initiale Freisetzungsrate der Arzneiform beträgt 3 % in 2 Stunden, die mittlere Freisetzungsrate 80 % in 14 Stunden.

## Beispiel 4

### Erfinderische Retardtablette

Zusammensetzung pro Tablette:

| GRANULAT: | |
|---|---|
| Ipsapironhydrochloridhydrat | 10,62 mg |
| HPC 300 mPa • s | 154,1 mg |
| Bernsteinsäure | 5,0 mg |

| NACHMISCHUNG: | |
|---|---|
| Magnesiumstearat | 0,3 mg |

| LACKHÜLLE: | |
|---|---|
| HPMC | 3,3 mg |
| PEG 4000 | 1,1 mg |
| Titandioxid | 1,1 mg |

Für eine Charge Tabletten werden eingewogen:

| | | |
|---|---|---|
| 1.<br>2. | HPC 300 mPa • s<br>Wasser | 1,5 g<br>348,8 g |
| HPC 300 mPa • s wird in Wasser gelöst. | | |
| 3. | Ipsapironhydrochlorid mikronisiert | 60,0 g |
| Ipsapironhydrochlorid wird in der HPC-M-Lösung suspendiert. Man erhält die Granulationsflüssigkeit. | | |
| 4.<br>5. | HPC 300 mPa • s<br>Bernsteinsäure | 923,1 g<br>30,0 g |
| Die Mischung von HPC 300 mPa • s und Bernsteinsäure wird im Wirbelschichtgranulator durch Einsprühen der Granulationsflüssigkeit granuliert. | | |
| 6. | Magnesiumstearat | 1,8 g |
| Das Magnesiumstearat wird mit dem getrockneten und gesiebten Granulat gemischt.<br>Die Mischung wird auf einer Tablettenmaschine zu Tabletten von 8 mm Durchmesser (12 mm Wölbungsradius) und von 170 mg Gewicht verpreßt. | | |
| 7.<br>8.<br>9. | Wasser 85-95 °C<br>Polyethylenglykol 4000<br>HPMC | 1270,0 g<br>30,0 g<br>90,0 g |
| PEG 4000 wird in heißem Wasser gelöst. Danach wird darin HPMC gelöst. | | |
| 10.<br>11. | Wasser<br>Titandioxid | 635,0 g<br>30,0 g |
| Titandioxid wird in Wasser suspendiert. Die Suspension wird mit der HPMC-PEG 4000-Lösung vereinigt und bis zur Erzielung des vorgeschriebenen Lackauftrages auf die Tabletten aufgesprüht. | | |

Die initiale Freisetzungsrate der Arzneiform beträgt 32 % in den ersten 2 Stunden, die mittlere Freisetzungsrate 80 % in 10 Stunden.

**Beispiel 5**

**Erfinderisches Retardpellet**

Zusammensetzung pro Kapsel:

PELLETKERN:

| | |
|---|---|
| Neutralpellets 0,85 - 1,00 mm | 40,00 mg |
| Ipsapironhydrochloridhydrat | 10,62 mg |
| Mikrofeines Kaliumhydrogentartrat | 10,00 mg |
| Hydroxypropylmethylcellulose | 5,00 mg |

DIFFUSIONSMEMBRAN:

| | |
|---|---|
| AQUACOAT® Trockensubstanz | 5,577 mg |
| Triacetin, eingesetzte Menge | 1,859 mg |

Hartgelatinekapsel

| | |
|---|---|
| Kapsel Gr. 4 | 39,00 mg |

Für eine Charge von 62.500 Retardkapseln werden eingewogen:

| | | |
|---|---|---|
| 1. | Neutralpellets 0,85 - 1,00 mm | 2.500,00 g |
| 2. | Ipsapiron HCl-hydrat | 663,75 g |
| 3. | Kaliumhydrogentartrat | 625,00 g |
| 4. | Methylhydroxypropylcellulose | 312,50 g |
| 5. | gereinigtes Wasser zur Herstellung der Beschichtungssuspension, das während des Prozesses verdampft wird. | 5.300,00 g |

Ipsapiron HCl und Kaliumhydrogentartrat werden in einer Teilmenge der angegebenen Menge Wasser suspendiert. Methylhydroxypropylcellulose wird in dem restlichen Wasser gelöst, beide Teilansätze vereinigt und vermischt. Diese Suspension wird in einer Wirbelschichtanlage durch einen Sprühprozess homogen auf den Neutralpellets verteilt.

Ferner werden eingewogen:

| | | |
|---|---|---|
| 6. | Ethylcellulose, wäßrige Dispersion 30 % (Aquacoat®) | 1.161,88 g |
| 7. | Triacetin | 116,19 g |
| 8. | gereinigtes Wasser zur Herstellung der Lacksuspension, das während des Prozesses verdampft wird. | 1.045,69 g |

Aquacoat® ECD 30 und Triacetin werden in der angegebenen Menge Wasser suspendiert. Mit dieser Lackdispersion werden die Wirkstoffpellets in einem Wirbelschichtprozess überzogen. Nach der Lackierung werden die Pellets im Trockenschrank 1 Stunden bei 60 °C getempert.

Als In-Prozess-Kontrolle werden der Gehalt und die Freisetzung bestimmt, so daß gegebenenfalls durch eine Nachlackierung das erfindungsgemäße Freisetzungsfenster erreicht werden kann.

Aus der Gehaltsbestimmung wird die endgültige Füllmenge der Kapseln bestimmt. Die 10 mg Ipsapiron HCl entsprechende Menge Pellets wird in Hartgelatinekapseln der Größe 4 abgefüllt.

Die initiale Freisetzungsrate der Arzneiform beträgt 22 % in den ersten 2 Stunden, die mittlere Freisetzungsrate 80 % in 5 Stunden.

## Beispiel 6

**Erfinderisches Retardpellet**

Zusammensetzung pro Kapsel:

PELLETKERN:

| | |
|---|---|
| Neutralpellets 0,85 - 1,00 mm | 40,00 mg |
| Ipsapironhydrochloridhydrat | 10,62 mg |
| Mikrofeines Kaliumhydrogentartrat | 10,00 mg |
| Hydroxypropylmethylcellulose | 5,00 mg |

DIFFUSIONSMEMBRAN:

| | |
|---|---|
| AQUACOAT® Trockensubstanz | 12,47 mg |
| Triacetin, eingesetzte Menge | 4,11 mg |

| | |
|---|---|
| Hartgelatinekapsel | |
| Kapsel Gr. 4 | 39,00 mg |

Die initiale Freisetzungsrate der Arzneiform beträgt 6,6 % in den ersten 2 Stunden, die mittlere Freisetzungsrate 80 % in 10 Stunden.

Die Herstellung erfolgt wie in Beispiel 5 beschrieben, jedoch wird das Aufsprühen der Aquacoat/Triacetin-Dispersion zweimal unterbrochen und die Pellets jeweils 1 Stunde bei 60 °C getrocknet.

## Beispiel 7

**Erfinderisches Retardpellet**

Zusammensetzung pro Kapsel:

PELLETKERN:

| | |
|---|---|
| Neutralpellets 0,85 - 1,00 mm | 120,00 mg |
| Ipsapironhydrochloridhydrat | 31,86 mg |
| Mikrofeines Kaliumhydrogentartrat | 30,00 mg |
| Hydroxypropylmethylcellulose | 15,00 mg |

DIFFUSIONSMEMBRAN:

| | |
|---|---|
| AQUACOAT® Trockensubstanz | 17,72 mg |
| Triacetin, eingesetzte Menge | 5,85 mg |

Hartgelatinekapsel

Die Herstellung erfolgt wie in Beispiel 5 beschrieben, jedoch werden die Pellets nach der Lackierung über 4 Stunden bei 65 °C getempert.

Die initiale Freisetzungsrate der Arzneiform beträgt 3,5 % in den ersten 2 Stunden, die mittlere Freisetzungsrate 80 % in 9 Stunden.

## Beispiel 8

### Erfinderisches Retardpellet

Zusammensetzung pro Kapsel:

PELLETKERN:

| | |
|---|---|
| Neutralpellets 0,85 - 1,00 mm | 80,00 mg |
| Ipsapironhydrochloridhydrat | 21,24 mg |
| Mikrofeines Kaliumhydrogentartrat | 20,00 mg |
| Hydroxypropylmethylcellulose | 10,00 mg |

DIFFUSIONSMEMBRAN:

| | |
|---|---|
| AQUACOAT® Trockensubstanz | 18,37 mg |
| Triacetin, eingesetzte Menge | 6,06 mg |

Hartgelatinekapsel

Die Herstellung erfolgt wie in Beispiel 5 beschrieben, jedoch werden die Pellets nach der Lackierung über 1 Stunde bei 60 °C getempert.

Die initiale Freisetzungsrate der Arzneiform beträgt 4,2 % in den ersten 2 Stunden, die mittlere Freisetzungsrate 80 % in 9 Stunden.

## Beispiel 9

**Erfinderisches Retardpellet**

Zusammensetzung pro Kapsel:

PELLETKERN:

| | |
|---|---|
| Neutralpellets 0,5 - 0,6 mm | 80,00 mg |
| Ipsapironhydrochloridhydrat | 21,24 mg |
| Kaliumhydrogentartrat | 20,00 mg |
| Hydroxypropylmethylcellulose | 5,00 mg |

DIFFUSIONSMEMBRAN:

| | |
|---|---|
| AQUACOAT® Trockensubstanz | 12,62 mg |
| Triacetin, eingesetzte Menge | 4,21 mg |

Hartgelatinekapsel

Die Herstellung erfolgt wie in Beispiel 5 beschrieben.

Die initiale Freisetzungsrate der Arzneiform beträgt 8 % in den ersten 2 Stunden, die mittlere Freisetzungsrate 80 % in 11 Stunden.

## Beispiel 10

**Erfinderisches Retardpellet**

Zusammensetzung pro Kapsel:

PELLETKERN:

| | |
|---|---|
| Neutralpellets 0,85 - 1,00 mm | 160,00 mg |
| Ipsapironhydrochloridhydrat | 42,48 mg |
| Kaliumhydrogentartrat | 40,00 mg |
| Hydroxypropylmethylcellulose | 20,00 mg |

DIFFUSIONSMEMBRAN:

| | |
|---|---|
| AQUACOAT® Trockensubstanz | 21,00 mg |
| Triacetin, eingesetzte Menge | 6,93 mg |

Hartgelatinekapsel

Die Herstellung erfolgt wie in Beispiel 5 beschrieben, jedoch werden die Pellets nach der Lackierung über 1 Stunde bei 65 °C in der Wirbelschicht getempert.

Die initiale Freisetzungsrate der Arzneiform beträgt 19 % in den ersten 2 Stunden, die mittlere Freisetzungsrate 80 % in 7 Stunden.

**Beispiel 11**

**Erfinderische Retardtablette**

Zusammensetzung pro Tablette:

| UNLACKIERTE TABLETTE: | |
|---|---|
| Ipsapironhydrochloridhydrat | 21,24 mg |
| HPC 8 mPa • s | 49,60 mg |
| HPC 300 mPa • s | 141,10 mg |
| Bernsteinsäure | 7,50 mg |
| Magnesiumstearat | 0,5 mg |

| LACKHÜLLE: | |
|---|---|
| HPMC | 3,60 mg |
| PEG 4000 | 1,20 mg |
| Titandioxid | 1,20 mg |

Es werden Tabletten von 226 mg mit einem Durchmesser von 9 mm und einem Wölbungsradius von 15 mm wie in Beispiel 4 beschrieben hergestellt. Die initiale Freisetzungsrate der Arzneiform beträgt 29 % in den ersten 2 Stunden und die mittlere Freisetzungsrate 80 % in 12 Stunden.

**Beispiel 12**

**Erfinderische Retardtablette**

Zusammensetzung pro Tablette:

| UNLACKIERTE TABLETTE: | |
|---|---|
| Ipsapironhydrochloridhydrat | 21,24 mg |
| HPC 300 mPa • s | 190,70 mg |
| Bernsteinsäure | 7,50 mg |
| Magnesiumstearat | 0,5 mg |

| LACKHÜLLE: | |
|---|---|
| HPMC | 3,60 mg |
| PEG 4000 | 1,20 mg |
| Titandioxid | 1,20 mg |

Es werden Tabletten von 226 mg mit einem Durchmesser von 9 mm und einem Wölbungsradius von 15 mm wie in Beispiel 4 beschrieben hergestellt. Die initiale Freisetzungsrate der Arzneiform beträgt 29 % in den ersten 2 Stunden und die mittlere Freisetzungsrate 80 % in 12 Stunden.

**Beispiel 13**

**Erfinderische Retardtablette**

Zusammensetzung pro Tablette:

| UNLACKIERTE TABLETTE: | |
|---|---|
| Ipsapironhydrochloridhydrat | 31,86 mg |
| HPC 8 mPa • s | 195,70 mg |
| HPC 300 mPa • s | 58,60 mg |
| Bernsteinsäure | 12,50 mg |
| Magnesiumstearat | 0,7 mg |

| LACKHÜLLE: | |
|---|---|
| HPMC | 4,20 mg |
| PEG 4000 | 1,40 mg |
| Titandioxid | 1,40 mg |

Es werden Tabletten von 307 mg mit einem Durchmesser von 10 mm und einem Wölbungsradius von 15 mm wie in Beispiel 4 beschrieben hergestellt. Die initiale Freisetzungsrate der Arzneiform beträgt 35 % in den ersten 2 Stunden und mittlere Freisetzungsrate 80 % in 7,5 Stunden.

**Beispiel 14**

**Erfinderische Retardtablette**

Zusammensetzung pro Tablette:

| UNLACKIERTE TABLETTE: | |
|---|---|
| Ipsapironhydrochloridhydrat | 31,86 mg |
| HPC 300 mPa • s | 254,90 mg |
| Bernsteinsäure | 12,50 mg |
| Magnesiumstearat | 0,7 mg |

| LACKHÜLLE: | |
|---|---|
| HPMC | 4,20 mg |
| PEG 4000 | 1,40 mg |
| Titandioxid | 1,40 mg |

Es werden Tabletten von 307 mg mit einem Durchmesser von 10 mm und einem Wölbungsradius von 15 mm wie in Beispiel 4 beschrieben hergestellt. Die initiale Freisetzungsrate der Arzneiform beträgt 28 % in den ersten 2 Stunden und die mittlere Freisetzungsrate 80 % in 13 Stunden.

**Beispiel 15**

**Erfinderisches Retardpellet**

Zusammensetzung pro Kapsel:

PELLETKERN:

| | |
|---|---|
| Neutralpellets 0,85 - 1,00 mm | 80,00 mg |
| Ipsapironhydrochloridhydrat | 21,24 mg |
| Mikrofeines Kaliumhydrogentartrat | 20,00 mg |
| Hydroxypropylmethylcellulose | 10,00 mg |

DIFFUSIONSMEMBRAN:

| | |
|---|---|
| AQUACOAT® Trockensubstanz | 18,36 mg |
| Triacetin, eingesetzte Menge | 6,00 mg |

Hartgelatinekapsel

Die Herstellung erfolgt wie in Beispiel 5 beschrieben, jedoch werden die Pellets nach der Lackierung über 2 Stunden bei 65 °C getempert.

Die initiale Freisetzungsrate der Arzneiform beträgt 5 % in den ersten 2 Stunden, die mittlere Freisetzungsrate 80 % in 12 Stunden.

Tabelle 1

| **Beispiel Nr.** | **1** | **2** | **3** | **4** | **5** | **6** |
|---|---|---|---|---|---|---|
| Art der Retardierung | keine | Erosionstablette | Diffusionspellet | Erosionstablette | Diffusionspellet | Diffusionspellet |
| Pellets: Beschichtungsmenge mit Diffusionslack | - | - | 23 % | - | 8,5 % | 19 % |
| Erosionstablette: Gehalt an Hydroxy-propylcellulose 300 mPa · s | - | 46,2 mg | - | 154,1 mg | - | - |
| Initiale Freisetzungs-rate* (nach 2 Stunden) | - | 40 %/2 h | 4 %/72 h | 32 %/2 h | 22 %/2 h | 7 %/2 h |
| Mittlere Freisetzungs-rate* | - | 80 %/6 h | 80 %/14 h | 80 %/10 h | 80 %/5 h | 80 %/10 h |
| Relative Bioverfüg-barkeit | 100 % | 78,3 % | 85,2 % | 107,3 % | 117,9 % | 124,4 % |

* USP paddle-Methode, 900 ml 0.1 N-Salzsäure, 75 Umdrehungen pro Minute

## Patentansprüche

1. Ipsapiron-Arzneizubereitung mit einer mittleren Freisetzungsrate zwischen 80 %/5 Stunden bis 80 %/13 Stunden und einer initialen Freisetzungsrate von weniger als 35 % Ipsapiron in den ersten beiden

Stunden der Freisetzung.

**2.** Ipsapiron-Arzneizubereitung nach Anspruch 1 in Form von diffusionskontrollierten Pettets oder von Tabletten.

**3.** Diffusionskontrollierte Pellets nach Anspruch 2 dadurch gekennzeichnet, daß sie aus mit einer Mischung aus Wirkstoff und Bindemittel überzogenen Neutralpellets bestehen, die mit einer Diffusionsschicht überzogen ist.

**4.** Diffusionskontrollierte Pellets nach Anspruch 2 bis 3 dadurch gekennzeichnet, daß die Diffusionsschicht aus einem üblichen Diffusionslack und einem üblichen Weichmacher besteht.

**5.** Diffusionskontrollierte Pellets nach Anspruch 2 bis 4 dadurch gekennzeichnet, daß als Bindemittel Hydroxypropylmethylcellulose, als Diffusionslack Aquacoat ® und als Weichmacher Triacetin verwendet wird.

**6.** Verfahren zur Herstellung von diffusionskontrollierten Pellets nach Anspruch 2 bis 5 dadurch gekennzeichnet, daß man den Wirkstoff gegebenenfalls mit Zusatzstoffen in Wasser suspendiert oder löst, mit einer Lösung eines Bindemittels verdickt, diese Suspension auf Neutralpellets aufzieht und diese beschichteten Pellets mit einer Mischung aus Diffusionslack und Weichmachern überzieht.

**7.** Tabletten nach Anspruch 2 enthaltend neben dem Wirkstoff und üblichen Tablettierhilfsmitteln wasserlösliche, hydrogelbildende Polymere.

**8.** Tablette nach Anspruch 7 dadurch gekennzeichnet, daß sie neben dem Wirkstoff mindestens 50 mg Hydroxypropylcellulose einer Viskosität von mindestens 150 mPa • s enthält.

**9.** Ipsapiron-Arzneizubereitungen nach Anspruch 1 bis 7 dadurch gekennzeichnet, daß sie zusätzlich eine physiologisch verträgliche Säure enthalten.

**10.** Verwendung von Ipsapiron-Arzneiformen nach Anspruch 1 bis 9 zur Behandlung von Angstzuständen und Depression.

Fig. 1

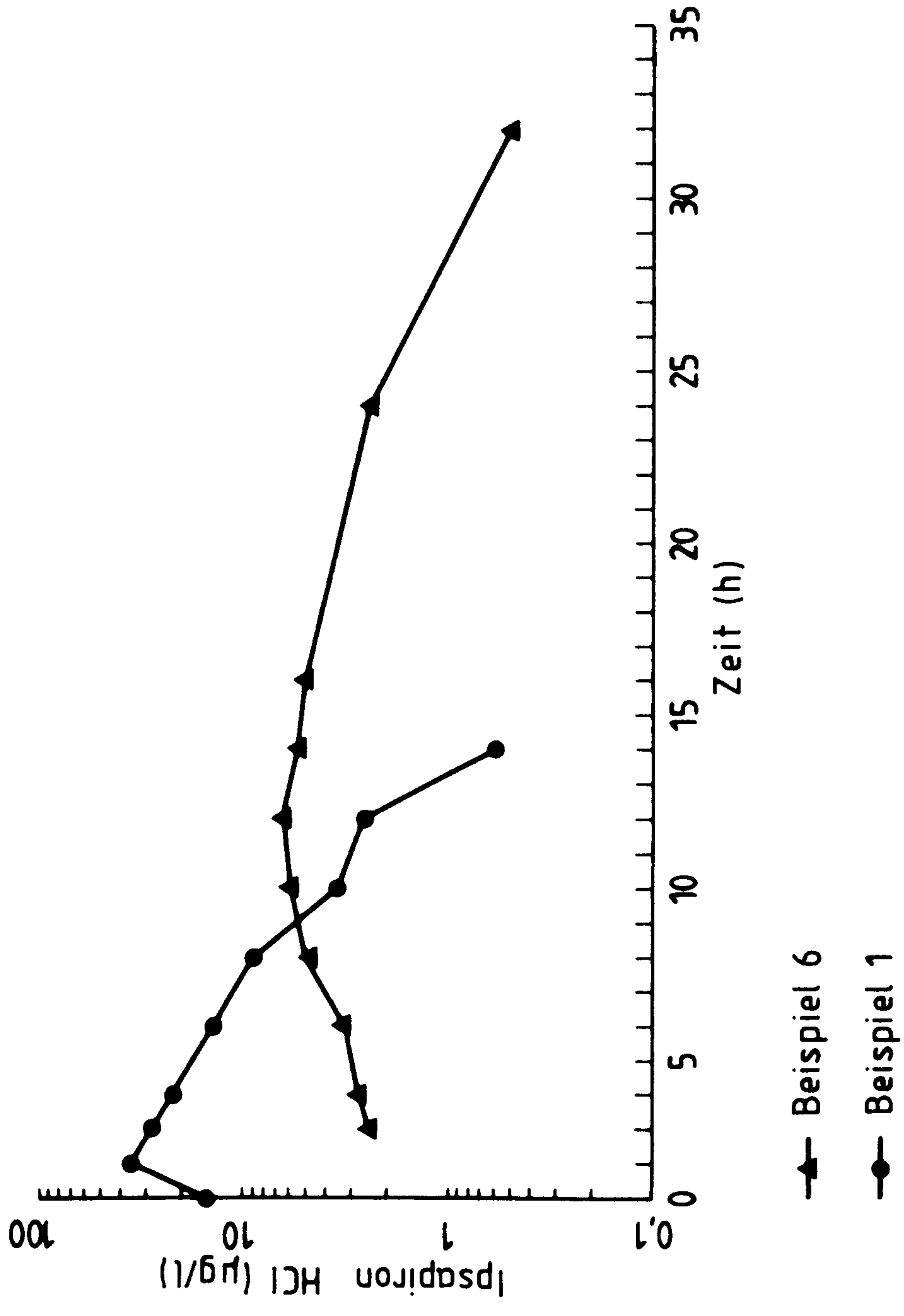
Ipsapiron HCl (µg/l)
100
10
1
0,1
0
5
10
15
20
25
30
35
Zeit (h)
Beispiel 6
Beispiel 1

Europäisches Patentamt

# EUROPÄISCHER RECHERCHENBERICHT

Nummer der Anmeldung
EP 94 10 8571

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | DATABASE WPI<br>Week 9304,<br>Derwent Publications Ltd., London, GB;<br>AN 93-032652 (04)<br>* Zusammenfassung *<br>& JP-A-04 360 826 (BAYER YAKUHIN KK) 14. Dezember 1992<br>--- | 1-10 | A61K31/505<br>A61K9/20<br>A61K9/28<br>A61K9/50 |
| Y | DATABASE WPI<br>Week 9225,<br>Derwent Publications Ltd., London, GB;<br>AN 92-200732 (25)<br>* Zusammenfassung *<br>& DD-A-297 767 (BAYER AG) 23. Januar 1992<br>--- | 1-10 | |
| Y | EP-A-0 386 440 (BAYER AG)<br>* Ansprüche *<br>----- | 1-10 | |
| | | | RECHERCHIERTE SACHGEBIETE (Int.Cl.5) |
| | | | A61K |

Der vorliegende Recherchenbericht wurde für alle Patentansprüche erstellt

| Recherchenort | Abschlußdatum der Recherche | Prüfer |
|---|---|---|
| DEN HAAG | 22. September 1994 | Scarponi, U |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument